# EUROPÄISCHE PATENTSCHRIFT

(11) **EP 2 314 270 B1**
(45) Veröffentlichungstag und Bekanntmachung des Hinweises auf die Patenterteilung: **03.07.2013**
(21) Anmeldenummer: 10186493.2
(22) Anmeldetag: 05.10.2010
(51) Int. Cl.: A61K 8/04, A61K 8/44, A61K 8/60, A61Q 5/02

(54) **Haarreinigungsprodukt**
Hair cleaning product
Produit de nettoyage des cheveux

(30) Priorität: 13.10.2009 DE 102009045606
(43) Veröffentlichungstag der Anmeldung: 27.04.2011
(73) Patentinhaber: Henkel AG & Co. KGaA, 40589 Düsseldorf (DE)
(72) Erfinder: Hippe, Thomas, 25482, Appen (DE); Kursawe, Petra, 22527, Hamburg (DE)

(56) Entgegenhaltungen:
- EP-A1- 1 266 652
- EP-A1- 1 493 802
- WO-A1-00/39273
- WO-A1-00/62755
- US-A- 5 346 639
- US-A- 5 560 918
- US-A1- 2002 045 670

## Beschreibung

Die vorliegende Erfindung liegt auf dem Gebiet der kosmetischen Mittel und betrifft ein Reinigungsprodukt, umfassend ein sensitives Reinigungsmittel und einen Spender.

Die Erfindung betrifft weiterhin ein kosmetisches Verfahren zur Haarreinigung.

Reinigungsmittel, beispielsweise kosmetische Reinigungsmittel für die Haut und die Haare wie flüssige Seifen, Shampoos, Duschbäder, Schaumbäder, Dusch- und Waschgele, müssen nicht nur ein gutes Reinigungsvermögen aufweisen, sondern sollen weiterhin für die Haut und die Schleimhäute gut verträglich sein und auch bei häufiger Anwendung nicht zu starker Entfettung oder Hauttrockenheit führen.

Aus diesem Grund wird seit vielen Jahren versucht, möglichst viele Haar- und Hautkonditioniermittel in die Reinigungsmittel einzuarbeiten.

Die Einarbeitung solcher Haut- und Haarkonditioniermittel in eine tensidische Reinigungsformulierung stellt die Hersteller solcher Zusammensetzungen aber immer wieder vor große Schwierigkeiten, denn insbesondere mineralische, natürliche oder synthetische Fett-, Wachs- und Ölkomponenten, die nachweislich einen pflegenden und konditionierenden Effekt auf der Haut und den Haaren hinterlassen, lassen sich nicht durch einfaches Einmischen in eine Tensidbasis einarbeiten.

Zur Stabilisierung der Haut- und Haarkonditioniermittel in einer Tensidbasis wurden zahlreiche Lösungsvorschläge veröffentlicht, die meist auf der Einarbeitung einer oder mehrerer Stabilisierungsmittel für die Haut- und Haarkonditioniermittel beruhen.

Die Einarbeitung dieser Stabilisierungsmittel ließen die Reinigungszusammensetzungen in ihrer Herstellung immer komplexer, aufwendiger und damit kostspieliger werden.

Ein weiterer Nachteil ist, dass durch die Vielzahl der zur Stabilisierung notwendigen Komponenten die Reinigungsmittel teilweise schlechter haut- und/oder kopfhautverträglich sind.

Es besteht damit weiterhin der Bedarf nach pflegenden und reinigenden Mitteln, die zeit- und kosteneffektiv hergestellt werden können. Die Mittel sollen mild auf der Haut/Kopfhaut wirken und keine zusätzlichen Stabilisierungsmittel enthalten.

Vorgeschlagen werden daher kosmetische Reinigungsmittel in einer neuen Applikationsform, die aufgrund einer speziellen Tensidmischung mild auf der Haut/Kopfhaut wirken.

Gegenstand der vorliegenden Erfindung ist ein sensitives kosmetisches Reinigungsprodukt, umfassend
(i) ein sensitives kosmetisches Reinigungsmittel, enthaltend
   a) mindestens ein mildes anionisches Tensid enthaltend Disodium Cocoylglutamate,
   b) mindestens ein amphoteres/zwitterionisches Tensid, und
   c) mindestens ein nichtionisches Tensid
   in einem Verhältnis der Tenside a), b) und c) im Bereich von (1-2): (0,5-1): (1-2), und
(ii) einen Spender zur Bevorratung und Abgabe des Reinigungsmittels (i), der mindestens einen Behälter zur Bevorratung des Reinigungsmittels, eine Abgabevorrichtung zur Abgabe des Reinigungsmittels in gesprühter Form und eine Fördereinrichtung zur Förderung des Reinigungsmittels vom Behälter zur Abgabevorrichtung aufweist.

Unter geeigneten Spendern (ii) für ein derartiges kosmetisches Reinigungsprodukt werden im Sinne der Erfindung Spenderausführungen verstanden, welche in der Lage sind, eine möglichst exakt dosierbare Abgabe des Reinigungsmittels in gesprühter Form zu bewirken. Dabei ist unter gesprühter Abgabeform eine Abgabe des Reinigungsmittels zu verstehen, bei der das Reinigungsmittel in Gestalt kleiner Reinigungsmitteltröpfchen bzw. als feiner Sprühnebel aus der Abgabevorrichtung austritt.

Als erste Gruppe derartiger Spender kommen grundsätzlich Spender mit zugehörigen Druckbehältern in Betracht, bei denen der das abzugebende Reinigungsmittel bevorratende Behälter unter Druck steht. Solche Druckbehälter erfordern in der Regel eine entsprechend steife Gestaltung des Behälters. Durch Betätigung einer mit dem Druckbehälter in Fluidverbindung stehenden Abgabevorrichtung kann dann das Reinigungsmittel dosiergenau abgegeben werden. Als Beispiel für solche Spender mit integrierter Druckquelle sind Druckbehälter zu nennen, die üblicherweise im Behälterinneren entweder einen geeigneten Druckspeicher aufweisen, z. B. einen mechanischen, oder aber ein Treibmittel enthalten und auf diesem Wege das Innere des Behälters unter Druck setzen. Derartige Druckbehälter verfügen üblicherweise über geeignete Ventilvorrichtungen zur Ausgabe des im Inneren des Druckbehälters befindlichen Reinigungsmittels während einer entsprechenden Ventilbetätigung. Solche Druckbehälter sind vor allem in Verbindung mit gasförmigen und/oder flüssigen Treibmitteln in Form von Aerosolspendern für unterschiedlichste kosmetische Anwendungen vorbekannt, z. B. Haarstylingspray, Haarfärbezubereitungen, Deospray, Rasierschaum/-gel etc..

Alternativ können prinzipiell auch Spenderausführungen mit Behälter und Abgabevorrichtung zum Einsatz kommen, welche neben einer Fördereinrichtung für das abzugebende Reinigungsmittel eine integrierte Energiequelle aufweisen, z. B. eine elektrische Energiequelle, insbesondere eine Batterie. Dabei dient die Energiequelle zum Antrieb der Fördereinrichtung, z. B. einer geeigneten Pumpe, mittels der das Reinigungsmittel aus dem Behälter zur Abgabevorrichtung gefördert werden kann. Auch hier wird die abzugebende Dosiermenge vorzugsweise über die Betätigungsintensität oder Betätigungsdauer bestimmt.

Als bevorzugte Spendervariante bietet sich erfindungsgemäß allerdings ein manuell durch den Nutzer zu betätigender Spender an. Bei derartigen Spendern wird die für die Mittelabgabe notwendige Energie als Folge einer manuellen Betätigung oder einer sonstigen Krafteinwirkung des Nutzers aufgebracht. Bei diesen Bauformen kann vorteilhaft auf eine zusätzliche Energie- oder Druckquelle, z. B. Treibmittel, verzichtet werden, was vor allem aus Kosten- sowie Nachhaltigkeitsgründen wünschenswert ist.

Eine erste Ausführungsform solch manueller Spender sind so genannte Quetsch- oder Squeeze-Spender, bei denen ein elastisch verformbarer Behälter zur Reinigungsmittelbevorratung durch äußerliche Krafteinwirkung des Nutzers eine Druckerhöhung im Behälterinneren bewirkt. Als Folge dieser Druckerhöhung wird das im Behälter befindliche Reinigungsmittel über die Fördereinrichtung zur Abgabevorrichtung gefördert und damit die Reinigungsmittelabgabe bewirkt. Die Fördereinrichtung ist im Falle derartiger Quetsch-Spender häufig als einfache Fluidverbindungsleitung ausgebildet. Bei entsprechender Gestaltung der Fördereinrichtung sind solche Quetsch-Spender in jeder beliebigen Positionierung verwendbar, insbesondere in Überkopfposition, d. h. bei untenliegender Abgabevorrichtung. Der elastische verformbare Behälter ist insbesondere tuben- oder flaschenförmig gestaltet und besitzt Abmessungen, die eine leichte Handhabung durch den Benutzer ermöglichen. Zur Gewährleistung der elastischen Verformbarkeit ist es notwendig die Vorratsbehälterwandung hinreichend flexibel bzw. reversibel verformbar zu gestalten. Dies wird gewährleistet durch eine anwendungsbezogen zielgerichtete Auslegung der Dicke der Vorratsbehälterwandung in Verbindung mit einer geeigneten Materialauswahl für die Vorratsbehälterwandung. Bevorzugt wird die Vorratsbehälterwandung eines entsprechenden Quetsch-Spenders aus einem Polyolefin, wie beispielsweise Polypropylen (PP), high density Polyethylen (HDPE), medium density Polyethylen (MDPE), low density Polyethylen (LDPE), linear low density Polyethylen (LLDPE), ausgeführt. Darunter ist Polypropylen (PP) bevorzugt geeignet. Darüber hinaus ist der Behälter zur besseren Handhabbarkeit derart bemessen, dass er vom Nutzer mit nur einer Hand gehalten und auch zur Reinigungsmittelabgabe gedrückt werden kann. Die grundsätzliche Funktionsweise derartiger Quetsch-Spender wird auch in den Dokumenten WO 2007/086730 A2/A3 sowie EP 1237660 B1 beschrieben. Ein für die erfindungsgemäßen Reinigungsprodukte geeigneter Quetsch-Spender kann auch entsprechend zu diesen Patentdokumenten ausgebildet sein. Insbesondere kann ein für die erfindungsgemäßen Reinigungsprodukte geeigneter Quetsch-Spender gemäß der Offenbarung von EP 1237660 B1 so ausgeführt werden, dass eine Verwendung des Quetsch-Spenders in im wesentlichen aufrechter Position als auch in Überkopfstellung möglich ist.

Eine weitere alternative Ausführungsform eines für die erfindungsgemäßen Reinigungsprodukte geeigneten manuellen Spenders ist der Pumpspender. Ein solcher Pumpspender umfasst in der Regel neben dem Behälter zu Bevorratung des abzugebenden Reinigungsmittels, eine Abgabevorrichtung zur dosiergenauen Reinigungsmittelabgabe sowie eine geeignete Fördereinrichtung zur Förderung des üblicherweise fließfähigen Reinigungsmittels aus dem Behälter zur Abgabevorrichtung. Die Fördereinrichtung ist bevorzugt als Förderpumpe ausgeführt, mittels der das Reinigungsmittel aus dem Behälter angesaugt und unter Zwischenschaltung der Pumpe zur Abgabevorrichtung gepumpt wird. Dabei ist die Fördereinrichtung im Falle des manuellen Spenders derart mit einer zugehörigen Betätigungsvorrichtung gekoppelt, dass bei Betätigung der Betätigungsvorrichtung durch den Nutzer die Fördereinrichtung entsprechend aktiviert wird. Um eine dosiergenaue Reinigungsmittelabgabe zu erreichen, ist die Aktivierung der Fördereinrichtung insbesondere von der Betätigungsintensität bzw. Betätigungsdauer abhängig. Die Betätigungsvorrichtung umfasst vorteilhaft einen Taster oder Druckschalter, über den der Nutzer die manuelle Krafteinwirkung zur Reinigungsmittelabgabe aufbringen kann. Derartige Pumpspender werden unter anderen auch in den Patentdokumenten EP 1472007 A1, DE 69916827 T2 beschrieben. Solche Pumpspender gestatten eine diskontinuierliche Reinigungsmittelabgabe, da bei jedem Pumphub lediglich eine definierte Reinigungsmittelmenge abgegeben wird.

Eine Sonderform des Pumpspenders zur kontinuierlichen Reinigungsmittelabgabe wird durch eine Bauform definiert, bei der die manuell vom Nutzer zu betätigende Pumpe nicht zur Förderung des Reinigungsmittels genutzt wird, sondern lediglich zum Druckaufbau im Behälter dient. Hierbei fördert die Pumpe bei Betätigung üblicherweise Umgebungsluft in den Behälter und erhöht dadurch dessen Innendruck. Als Folge einer manuellen Mehrfachbetätigung der Pumpe nach Art einer Luftpumpe stellt sich somit ein Behälterinnendruck ein, der eine kontinuierliche Reinigungsmittelabgabe über einen längeren Zeitraum zulässt. Die dosiergenaue Reinigungsmittelabgabe erfolgt hier üblicherweise über die Betätigungsdauer des zugehörigen Betätigungselements.

Für alle vorbeschriebenen Spenderausführungen wird die Abgabekonsistenz des Reinigungsmittels durch die spezifische Abgabevorrichtung bestimmt.

Zur Sprühabgabe weist die Abgabevorrichtung vorteilhaft eine geeignete Austrittsdüse auf, welche die gewünschte Konsistenz in Form feiner Reinigungsmitteltröpfchen oder als Reinigungsmittel-Sprühnebel bereitstellt. In Abhängigkeit von der jeweiligen Austrittsmenge an Reinigungsmittel bzw. der Austrittsgeschwindigkeit des Reinigungsmittels kann die Austrittsdüse für die gewünschte Sprühkonsistenz bei der Reinigungsmittelabgabe gezielt ausgelegt werden. Ergänzend kann es sinnvoll sein, die Abgabevorrichtung mit einer Mischvorrichtung auszustatten, welche während der Reinigungsmittelabgabe das fließfähige bzw. viskose Reinigungsmittel mit Gas, vorzugsweise Umgebungsluft, mischt, um die gewünscht Sprühkonsistenz einzustellen. Durch Variation der Mischvorrichtung und/oder der Austrittsdüse kann somit gezielt die Abgabekonsistenz des Reinigungsmittels beeinflusst werden.

Darüber hinaus kann es sinnvoll sein, die vorbeschriebenen Spender mit mehreren Behältern für jeweils unterschiedliche Reinigungsmittelkomponenten auszustatten. Diese bietet sich vor allem bei miteinander unverträglichen Reinigungsmittelkomponenten an. Erst während einer Produktanwendung, d. h. während einer Spenderbetätigung werden dann die einzelnen Reinigungsmittelkomponenten unmittelbar vor der Reinigungsmittelabgabe miteinander vermischt. Besonders bevorzugte Reinigungsprodukte sind solche, die das Reinigungsmittel in einem Pumpspender, wie sie beispielsweise von der Firma Rexam SMT oder Seaquist erhältlich sind, enthalten.

Insbesondere geeignet für die Erzeugung eines feinen Sprühnebels sind die Sprühbehälter Y150 Airless oder SP32 Airless der Firme Rexam oder der Pumpzerstäuber PZ 2 / 190 DL 20/410 der Firma Seaquist.

Die Reinigungsmittel (i), die für die Applikation aus einem zuvor beschriebenen Spender (ii) geeignet sind, sind vorzugsweise niedrigviskos, damit durch Schütteln eine leichte und schnelle Durchmischung der Inhaltsstoffe sowie der Sprühvorgang - d.h. die Erzeugung des Sprühnebels - möglich ist. Unter "niedrigviskos" wird erfindungsgemäß eine Viskosität von maximal 1000 mPas, bevorzugt von maximal 750 mPas und insbesondere von maximal 500 mPas (gemessen mit einem Haake-Viskosimeter NV bei 20°C und 8 UpM) verstanden.

Zur Erzielung der zuvor genannten Viskosität sind die Reinigungsmittel (i) in einer bevorzugten Ausführungsform frei von polymeren Verdickungsmitteln.

Die Tenside a) bis c) werden in dem vorgenannten Verhältnis in den Reinigungsmitteln (i) eingesetzt. Dabei entfallen auf die einzelnen Tensidbestandteile a), b) und c) bevorzugt Mengen von
a) 0,1 bis 12,5 Gew.-%, mehr bevorzugt 0,5 bis 10 Gew.-% und insbesondere 1 bis 7,5 Gew.-%,
b) 0,1 bis 10 Gew.-%, mehr bevorzugt 0,25 bis 7,5 Gew.-% und insbesondere 0,5 bis 5 Gew.-%,
c) 0,1 bis 15 Gew.-%, mehr bevorzugt 0,5 bis 12,5 Gew.-% und insbesondere 1 bis 10 Gew.-%;
wobei die Mengenangaben sich jeweils auf das Gesamtgewicht des Reinigungsmittels (i) beziehen.

Als Komponente a) wird das unter der INCI-Bezeichnung Disodium Cocoyl Glutamate bekannte anionische Tensid eingesetzt.

Bevorzugt sind die unter den Handelsnamen Aminsoft® CS 11, Plantapon® ACG 35 und Plantapon® ACG 50 vertriebenen Produkte.

Als zweite essentielle Tensidkomponente wird ein amphoteres und/oder zwitterionisches Tensid b) in den Reinigungsmitteln (i) eingesetzt, das bevorzugt aus der Gruppe der
- N-Alkylglycine,
- N-Alkylpropionsäuren,
- N-Alkylaminobuttersäuren,
- N-Alkyliminodipropionsäuren,
- N-Hydroxyethyl-N-alkylamidopropylglycine,
- N-Alkyltaurine,
- N-Alkylsarcosine,
- 2-Alkylaminopropionsäuren mit jeweils etwa 8 bis 24 C-Atomen in der Alkylgruppe,
- Alkylaminoessigsäuren mit jeweils etwa 8 bis 24 C-Atomen in der Alkylgruppe,
- N-Kokosalkylaminopropionaten,
- Kokosacylaminoethylaminopropionaten
- C₁₂-C₁₈-Acylsarcosinen,
- N-Alkyl-N,N-dimethylammoniumglycinaten, z.B. Kokosalkyldimethylammoniumglycinat,
- N-Acyl-aminopropyl-N,N-dimethylammoniumglycinaten, beispielsweise Kokosacylaminopropyl-dimethylammoniumglycinat,
- 2-Alkyl-3-carboxymethyl-3-hydroxyethyl-imidazolinen mit jeweils 8 bis 18 C-Atomen in der Alkyl- oder Acylgruppe
- Kokosacylaminoethylhydroxyethylcarboxymethylglycinat
- den unter der INCI-Bezeichnung Cocamidopropyl Betain bekannten Verbindungen,
- den unter der INCI-Bezeichnung Disodium Cocoamphodiacetate bekannten Verbindungen, ausgewählt sein kann.

Neben den amphoteren/zwitterionischen Tensiden b) können den Reinigungsmitteln (i) auch weitere amphotere/zwitterionische Verbindungen zugesetzt werden. Diese können bevorzugt ausgewählt sein aus Verbindungen der Formel (II) 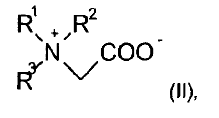 in der die Reste R¹ bis R³ unabhängig voneinander für C₁-C₄-Alkylgruppen oder Hydroxyalkylgruppen stehen. Bevorzugt stehen die Reste R¹ bis R³ für gleiche Reste Methyl- oder Ethyl und insbesondere bevorzugte stehen alle drei Reste R¹ bis R³ für Methylreste.

Eine besonders geeignete Verbindung der Formel (II) ist das unter der INCI-Bezeichnung "Betaine" vertriebene Produkt der Formel (II), beispielsweise das unter dem Handelsnamen Tego Natural Betaine^{®} vertriebene Produkt der Firma Goldschmidt (Evonik-Degussa). Tego Natural Betaine^{®} ist insbesondere geeignet, weil es überwiegend natürlichen Ursprungs ist und ausgezeichnete Moisturizing- und Hautpflegeeigenschaften aufweist.

Geeignete nichtionische Tenside c) können ausgewählt sein aus:
- Polyolfettsäureestern, wie beispielsweise das Handelsprodukt Hydageno^{®} HSP (Cognis) oder Sovermol - Typen (Cognis),
- Aminoxiden,
- Hydroxymischethern,
- Sorbitanfettsäureestern,
- Zuckerfettsäureestern,
- Fettsäure-N-alkylglucamiden und
- Alkylpolyglucosiden.

Geeignete Alkylpolyglucoside entsprechen der Formel (III)

R¹O-[G]ₚ (III)

in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht. Die Alkyl- und/oder Alkenyloligoglycoside können sich von Aldosen bzw. Ketosen mit 5 oder 6 Kohlenstoffatomen, vorzugsweise der Glucose ableiten. Die bevorzugten Alkyl- und/oder Alkenyloligoglycoside sind somit Alkyl- und/oder Alkenyloligoglucoside. Die Indexzahl p in der allgemeinen Formel (III) gibt den Oligomerisierungsgrad (DP), d.h. die Verteilung von Mono- und Oligoglycosiden anund steht für eine Zahl zwischen 1 und 10. Während p in einer gegebenen Verbindung stets ganzzahlig sein muss und hier vor allem die Werte p = 1 bis 6 annehmen kann, ist der Wert p für ein bestimmtes Alkyloligoglycosid eine analytisch ermittelte rechnerische Größe, die meistens eine gebrochene Zahl darstellt. Vorzugsweise werden Alkyl- und/oder Alkenyloligoglycoside mit einem mittleren Oligomerisierungsgrad p von 1,1 bis 3,0 eingesetzt. Aus anwendungstechnischer Sicht sind solche Alkyl- und/oder Alkenyloligoglycoside bevorzugt, deren Oligomerisierungsgrad kleiner als 1,7 ist und insbesondere zwischen 1,2 und 1,7 liegt. Der Alkyl- bzw. Alkenylrest R¹ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen. Bevorzugt sind Alkyloligoglucoside der Kettenlänge C₈-C₁₀ (DP = 1-3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1-3). Der Alkyl- bzw. Alkenylrest R¹ kann sich ferner auch von primären Alkoholen mit 12 bis 22, vorzugsweise 12 bis 14 Kohlenstoffatomen ableiten. Typische Beispiele sind Laurylalkohol, Myristylakohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucyalkohol, Brassidylalkohol sowie deren technsiche Gemische.

Ganz besonders bevorzugt sind Alkyloligoglucoside auf der Basis von gehärtetem C_{12/14}-Kokosalkohol mit einem DP von 1-3, wie sie beispielsweise unter der INCI-Bezeichnung "Coco-Glucoside" im Handel erhältlich sind.

Eine besonders bevorzugte Tensidkombination a), b) und c) umfasst die unter den INCI-Bezeichnungen bekannten Tenside
- Disodium Cocoyl Glutamate,
- ein Sodium Cocoamphodiacetate und
- Coco Glucoside
in dem vorgenannten Verhältnis von (1-2): (0,5-1) : (1-2).

In einer bevorzugten Ausführungsform der Erfindung sind die Reinigungsmittel (i) im Wesentlichen frei von weiteren Tensiden.

Unter "im wesentlichen frei" ist ein maximaler Gehalt von 4 Gew.-%, bevorzugt von 3 Gew.-%, mehr bevorzugt von 2,5 Gew.-% und insbesondere von 2 Gew.-% zu verstehen, wobei sich der maximale Gehalt weiterer Tenside auf das Gesamtgewicht der Reinigungsmittel (i) bezieht. Nachteilig an sensitiven kosmetischen Reinigungsformulierungen war bisher, dass sie meist auf anionischen (wenn auch milden) Sulfattensiden basierten. Der Grund dafür ist, dass Verbraucher Shampoos und/oder Reinigungszusammensetzungen erwarten, die einen cremigen, feinporigen Schaum bilden, der sich angenehm anfühlt, und der sich leicht auf der Haut und den Haaren verteilen lässt. Diese Anforderungen an den Schaum ließen sich bislang nur aber durch einen Gehalt an anionischen Sufattensiden erzielen.

Durch die spezielle Tensidkombination in den Reinigungsmitteln (i) konnten diese Anforderungen an die Schaumqualität ebenfalls erzielt werden, weshalb in einer weiteren bevorzugten Ausführungsform der Erfindung auf anionische Sulfattenside verzichtet werden kann.

Bevorzugte Reinigungsmittel (i) sind daher frei von anionischen Sulfattensiden.

Weiterhin neigen manche PEG-Verbindungen als Emulgator für Öl und Wasser dazu, die Haut für Schadstoffe durchlässiger zu machen, weshalb in einer weiteren bevorzugten Ausführungsform die Reinigungszusammensetzungen (i) daher auch frei von PEG-Verbindungen formuliert werden. Unter "PEG-Verbindungen" werden:
1) Verbindungen der Formel (IV)

   H-(O-CH₂CH₂)ₙ-OH (IV),

   in der n für ganze Zahlen zwischen 1 und 100.000 steht,
2) Verbindungen der Formel (V)

   H-(O-CH(CH₃)CH₂)ₙ-OH (V),

   in der n für ganze Zahlen zwischen 1 und 100.000 steht,
3) Verbindungen der Formel (VI)

   R-(O-CH₂CH₂)ₙ-OH (VI),

   in der R für einen Alkyl- oder Alkenylrest und n für Zahlen zwischen 1 und 10.000 steht,
4) Verbindungen der Formel (VII)

   R-(O-CH₂CH₂)ₙ-OSO₃H (VII),

   in der R für einen Alkyl- oder Alkenylrest und n für Zahlen zwischen 1 und 10.000 steht, und
5) Verbindungen, die Gruppierungen der Formel (VIII) enthalten,

   -(O-CH₂CH₂)ₙ- (VII),

   in der n für ganze Zahlen zwischen 2 und 100.000 steht,
verstanden.

Neben der Reinigung ist auch die Pflege bzw. die Konditionierung der Haut/Kopfhaut und der Haare ein Erfordernis, das die Reinigungsmittel (i) erfüllen sollen.

Zu diesem Zweck können sie in einer weiteren bevorzugten Ausführungsform - bezogen auf ihr Gesamtgewicht - 0,01 bis 3 Gew.-% mindestens eines kationischen Polymeren enthalten.

Unter geeigneten kationischen Polymeren sind Polymere zu verstehen, welche in der Haupt- und/oder Seitenkette Gruppen aufweisen, welche "temporär" oder "permanent" kationisch sein können. Als "permanent kationisch" werden solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C₁₋₄-Kohlenwasserstoffgruppe an eine aus Acrylsäure, Methacrylsäure oder deren Derivaten aufgebaute Polymerhauptkette gebunden sind, haben sich als besonders geeignet erwiesen.

Homopolymere, die eine Gruppierung der allgemeinen Formel (IX), 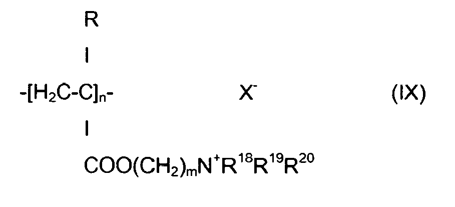 in der R¹⁷= -H oder -CH₃ ist, R¹⁸, R¹⁹ und R²⁰ unabhängig voneinander ausgewählt sind aus C₁₋₄-Alkyl-, -Alkenyl- oder -Hydroxyalkylgruppen, m = 1, 2, 3 oder 4, n eine natürliche Zahl und X⁻ ein physiologisch verträgliches organisches oder anorganisches Anion ist, sowie Copolymere, bestehend im wesentlichen aus den in Formel (IX) aufgeführten Monomereinheiten sowie nichtionogenen Monomereinheiten, sind besonders bevorzugte kationische Polymere. Im Rahmen dieser Polymere sind diejenigen bevorzugt, für die mindestens eine der folgenden Bedingungen gilt:
- R¹⁷ steht für eine Methylgruppe
- R¹⁸, R¹⁹ und R²⁰ stehen für Methylgruppen
- m hat den Wert 2.

Als physiologisch verträgliches Gegenionen X⁻ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein geeignetes Homopolymer ist das, gewünschtenfalls vernetzte,

Poly(methacryloyloxyethyltrimethylammoniumchlorid) mit der INCI-Bezeichnung Polyquaternium-37. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Das Homopolymer wird bevorzugt in Form einer nicht-wäßrigen Polymerdispersion, die einen Polymeranteil nicht unter 30 Gew.-% aufweisen sollte, eingesetzt. Solche Polymerdispersionen sind unter den Bezeichnungen Salcare^{®} SC 95 (ca. 50 % Polymeranteil, weitere Komponenten: Mineralöl (INCI-Bezeichnung: Mineral Oil) und Tridecyl-polyoxypropylen-polyoxyethylen-ether (INCI-Bezeichnung: PPG-1-Trideceth-6)) und Salcare^{®} SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylen-ether (INCI-Bezeichnung: PPG-1-Trideceth-6)) im Handel erhältlich.

Copolymere mit Monomereinheiten gemäß Formel (IX) enthalten als nichtionogene Monomereinheiten bevorzugt Acrylamid, Methacrylamid, Acrylsäure-C₁₋₄-alkylester und Methacrylsäure-C₁₋₄-alkylester. Unter diesen nichtionogenen Monomeren ist das Acrylamid besonders bevorzugt. Auch diese Copolymere können, wie im Falle der Homopolymere oben beschrieben, vernetzt sein. Ein bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwäßrige Polymerdispersion unter der Bezeichnung Salcare^{®} SC 92 erhältlich.

Weitere bevorzugte kationische Polymere sind beispielsweise
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate,
- hydrophob modifizierte Cellulosederivate, beispielsweise die unter dem Handelsnamen SoftCat^{®} vertriebenen kationischen Polymere,
- kationische Alkylpolyglycoside,
- kationiserter Honig, beispielsweise das Handelsprodukt Honeyquat^{®} 50,
- kationische Guar-Derivate, wie insbesondere die unter den Handelsnamen Cosmedia^{®}Guar und Jaguar^{®} vertriebenen Produkte,
- polymere Dimethyldiallylammoniumsalze und deren Copolymere mit Estern und Amiden von Acrylsäure und Methacrylsäure. Die unter den Bezeichnungen Merquat^{®}100 (Poly(dimethyldiallylammoniumchlorid)) und Merquat^{®}550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat^{®}734 und Gafquat^{®}755 im Handel erhältlich,
- Vinylpyrrolidon-Vinylimidazoliummethochlorid-Copolymere, wie sie unter den Bezeichnungen Luviquat^{®} FC 370, FC 550, FC 905 und HM 552 angeboten werden,
- quaternierter Polyvinylalkohol,
   sowie die unter den Bezeichnungen
- Polyquaternium 2, Polyquaternium 17, Polyquaternium 18, Polyquaternium 27, Polyquaternium 67, Polyquaternium 74 und Polyquaternium 89 bekannten Polymere.

Gleichfalls als kationische Polymere eingesetzt werden können die unter den Bezeichnungen Polyquaternium-24 (Handelsprodukt z. B. Quatrisoft^{®} LM 200), bekannten Polymere. Ebenfalls verwendbar sind die Copolymere des Vinylpyrrolidons, wie sie als Handelsprodukte Copolymer 845 (Hersteller: ISP), Gaffix^{®} VC 713 (Hersteller: ISP), Gafquat^{®}ASCP 1011, Gafquat^{®}HS 110, Luviquat^{®}8155 und Luviquat^{®} MS 370 erhältlich sind.

Weitere kationische Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen^{®} CMF, Hydagen^{®} HCMF, Kytamer^{®} PC und Chitolam^{®} NB/101 im Handel frei verfügbar sind. Chitosane sind deacetylierte Chitine, die in unterschiedlichen Deacetylierungsgraden und unterschiedlichen Abbaugraden (Molekulargewichten) im Handel erhältlich sind.

In einer besonders bevorzugten Ausführungsform enthalten die Reinigungsmittel (i) mindestens ein kationisches Cellulosepolymer, ein kationisches Guarpolymer und/oder ein kationisches Polymer auf Acrylsäure(derivat)basis, die insbesondere ausgewählt sind unter den unter den INCI-Bezeichnung bekannten Jaguar^{®}- oder N-Hance^{®}-Polymeren, Polyquaternium-6, Polyquaternium-7, Polyquaternium-10, Polyquaternium-67, Polyquaternium 74 und/oder Polyquaternium 89.

Eine weitere bevorzugte haut- und/oder haarkonditionierende Komponente, die in den Reinigungsmitteln (i) eingesetzt werden kann, ist mindestens eine Ölkomponente, die bevorzugt niedrigviskos ist, damit eine rasche Durchmischung durch Schütteln des Mittels vor der Applikation gewährleistet ist. Unter "niedrigviskos" wird eine Viskosität von maximal 1000 mPas (gemessen mit einem Haake -Viskosimeter NV bei 25°C und 8 UpM) verstanden.

Die Ölkomponente kann ausgewählt sein aus mineralischen, natürlichen oder synthetischen Ölkomponenten wie Petrolatum, Paraffinen, Silikonen, Fettalkoholen, Fettsäuren, Fettsäureestern sowie natürlichen Ölen pflanzlichen und tierischen Ursprungs. Sie wird in den Reinigungsmitteln (i) - bezogen auf ihr Gesamtgewicht - bevorzugt in einer Menge von 0,005 bis 10 Gew.-%, mehr bevorzugt von 0,01 bis 5 Gew.-% und insbesondere von 0,05 bis 3 Gew.-% eingesetzt.

Unter dem Begriff Silikonöle versteht der Fachmann mehrere Strukturen siliciumorganischer Verbindungen, die bevorzugt aus mindestens einem Vertreter der Gruppe siliciumorganischer Verbindungen ausgewählt wird, die gebildet wird aus:
(i) Polyalkylsiloxanen, Polyarylsiloxanen, Polyalkylarylsiloxanen, die flüchtig oder nicht flüchtig, geradkettig, verzweigt oder cyclisch, vernetzt oder nicht vernetzt sind;
(ii) Polysiloxanen, die in ihrer allgemeinen Struktur eine oder mehrere organofunktionelle Gruppen enthalten, die ausgewählt sind unter:
   a) substituierten oder unsubstituierten aminierten Gruppen;
   b) (per)fluorierten Gruppen;
   c) Thiolgruppen;
   d) Carboxylatgruppen;
   e) hydroxylierten Gruppen;
   f) alkoxylierten Gruppen;
   g) Acyloxyalkylgruppen;
   h) amphoteren Gruppen;
   i) Bisulfitgruppen;
   j) Hydroxyacylaminogruppen;
   k) Carboxygruppen;
   l) Sulfonsäuregruppen; und
   m) Sulfat- oder Thiosulfatgruppen;
(iii) linearen Polysiloxan(A)- Polyoxyalkylen(B)- Blockcopoylmeren vom Typ (A-B)ₙ mit n > 3;
(iv) gepfropften Silikonpolymeren mit nicht silikonhaltigem, organischen Grundgerüst, die aus einer organischen Hauptkette bestehen, welche aus organischen Monomeren gebildet wird, die kein Silikon enthalten, auf die in der Kette sowie gegebenenfalls an mindestens einem Kettenende mindestens ein Polysiloxanmakromer gepfropft wurde;
(v) gepfropften Silikonpolymeren mit Polysiloxan-Grundgerüst, auf das nicht silikonhaltige, organische Monomere gepfropft wurden, die eine Polysiloxan-Hauptkette aufweisen, auf die in der Kette sowie gegebenenfalls an mindestens einem ihrer Enden mindestens ein organisches Makromer gepfropft wurde, das kein Silikon enthält, wie beispielsweise das unter der INCI-Bezeichnung Bis-PEG/PPG-20/20 Dimethicone vertriebene Handelsprodukt Abil B 8832 der Firma Degussa;
(vi) oder deren Gemischen.

Besonders bevorzugte konditionierende Silikone sind die Dimethicone, Amodimethicone oder Dimethiconole.

Als Fettsäuren können eingesetzt werden lineare und/oder verzweigte, gesättigte und/oder ungesättigte Fettsäuren mit 6 - 30 Kohlenstoffatomen. Bevorzugt sind Fettsäuren mit 10 - 22 Kohlenstoffatomen. Hierunter wären beispielsweise zu nennen die Isostearinsäuren, wie die Handelsprodukte Emersol® 871 und Emersol® 875, und Isopalmitinsäuren wie das Handelsprodukt Edenor® IP 95, sowie alle weiteren unter den Handelsbezeichnungen Edenor® (Cognis) vertriebenen Fettsäuren. Weitere typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Besonders bevorzugt sind üblicherweise die Fettsäureschnitte, welche aus Cocosöl oder Palmöl erhältlich sind; insbesondere bevorzugt ist Stearinsäure.

Als Fettalkohole können eingesetzt werden gesättigte, ein- oder mehrfach ungesättigte, verzweigte oder unverzweigte Fettalkohole mit C₆-C₃₀-, bevorzugt C₁₀-C₂₂- und ganz besonders bevorzugt C₁₂-C₂₂- Kohlenstoffatomen. Einsetzbar sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen. Solche Substanzen sind beispielsweise unter den Bezeichnungen Stenol®, z.B. Stenol® 1618 oder Lanette®, z.B. Lanette® O oder Lorol®, z.B. Lorol® C8, Lorol® C14, Lorol® C18, Lorol® C8-18, HD-Ocenol®, Crodacol®, z.B. Crodacol® CS, Novol®, Eutanol® G, Guerbitol® 16, Guerbitol® 18, Guerbitol® 20, Isofol® 12, Isofol® 16, Isofol® 24, Isofol® 36, Isocarb® 12, Isocarb® 16 oder Isocarb® 24 käuflich zu erwerben. Selbstverständlich können erfindungsgemäß auch Wollwachsalkohole, wie sie beispielsweise unter den Bezeichnungen Corona®, White Swan®, Coronet® oder Fluilan® käuflich zu erwerben sind, eingesetzt werden.

Als natürliche oder synthetische Wachse können feste Paraffine oder Isoparaffine, Carnaubawachse, Bienenwachse, Candelillawachse, Ozokerite, Ceresin, Walrat, Sonnenblumenwachs, Fruchtwachse wie beispielsweise Apfelwachs oder Citruswachs, Microwachse aus PE- oder PP eingesetzt werden. Derartige Wachse sind beispielsweise erhältlich über die Fa. Kahl & Co., Trittau.

Zu den natürlichen und synthetischen kosmetischen Ölkörpern sind beispielsweise zu zählen:
- flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol® S) und Di-n-octylether (Cetiol® OE) können bevorzugt sein.
- Esteröle. Unter Esterölen sind zu verstehen die Ester von C₆-C₃₀ - Fettsäuren mit C₂-C₃₀ - Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen. Besonders bevorzugt sind Isopropylmyristat (Rilanit® IPM), Isononansäure-C₁₆-₁₈-alkylester (Cetiol® SN), 2-Ethylhexylpalmitat (Cegesoft® 24), Stearinsäure-2-ethylhexylester (Cetiol® 868), Cetyloleat, Glycerintricaprylat, Kokosfettalkohol-caprinat/-caprylat (Cetiol® LC), n-Butylstearat, Oleylerucat (Cetiol® J 600), Isopropylpalmitat (Rilanit® IPP), Oleyl Oleate (Cetiol®), Laurinsäurehexylester (Cetiol® A), Di-n-butyladipat (Cetiol® B), Myristylmyristat (Cetiol® MM), Cetearyl Isononanoate (Cetiol® SN), Olsäuredecylester (Cetiol® V).
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-di-isotridecanoat, Propylenglykol-di(2-ethylhexanoat), Propylenglykol-di-isostearat, Propylenglykol-dipelargonat, Butandiol-di-isostearat, Neopentylglykoldicaprylat,
- symmetrische, unsymmetrische oder cyclische Ester der Kohlensäure mit Fettalkoholen, Glycerincarbonat oder Dicaprylylcarbonat (Cetiol® CC),
- Trifettsäureester von gesättigten und/oder ungesättigten linearen und/oder verzweigten Fettsäuren mit Glycerin,

In einer besonders bevorzugten Ausführungsform der Erfindung wird als Ölkomponente ein pflanzliches Öl eingesetzt.

Als natürliche Öle kommen beispielsweise Amaranthsamenöl, Aprikosenkernöl, Arganöl, Avocadoöl, Babassuöl, Baumwollsaatöl, Borretschsamenöl, Camelinaöl, Distelöl, Erdnußöl, Granatapfelkernöl, Grapefruitsamenöl, Hanföl, Hagebuttenkernöl, Haselnussöl, Holundersamenöl, Johannisbeersamenöl, Jojobaöl, Kakaobutter, Leinöl, Macadamianussöl, Maiskeimöl, Mandelöl, Marulaöl, Nachtkerzenöl, Olivenöl, Palmöl, Pfirsichkernöl, Rapsöl, Reisöl, Sanddornfruchtfleischöl, Sanddornkernöl, Sesamöl, Sheabutter, Sojaöl, Sonnenblumenöl, Traubenkernöl, Walnußöl oder Wildrosenöl in Frage.

Besonders bevorzugt sind Avocadoöl, Aprikosenkernöl, Hagebuttenkernöl, Jojobaöl, Kakaobutter, Mandelöl, Olivenöl, Pfirsichkernöl, Sheabutter, Sonnenblumenöl und Traubenkernöl.

Neben den zwingenden und den zuvor genannten fakultativen Inhaltsstoffen können die Reinigungsmittel (i) zur weiteren Steigerung der haut- und/oder haarkonditionierenden Eigenschaften eine Reihe weiterer fakultativer Komponenten enthalten. Zu diesen zählen beispielsweise die Proteinhydrolysate.

Geeignete Proteinhydrolysate sind Produktgemische, die durch sauer, basisch oder enzymatisch katalysierten Abbau von Proteinen (Eiweißen) erhalten werden.

Es können Proteinhydrolysate sowohl pflanzlichen als auch tierischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan^{®} (Cognis), Promois^{®} (Interorgana), Collapuron^{®} (Cognis), Nutrilan^{®} (Cognis), Gelita-Sol^{®} (Deutsche Gelatine Fabriken Stoess & Co), Lexein^{®} (Inolex) und Kerasol^{®} (Croda) vertrieben.

Bevorzugt sind Proteinhydrolysate pflanzlichen Ursprungs, z. B. Soja-, Mandel-, Reis-, Erbsen-, Kartoffel- und Weizenproteinhydrolysate. Solche Produkte sind beispielsweise unter den Warenzeichen Gluadin^{®} (Cognis), DiaMin^{®} (Diamalt), Lexein^{®} (Inolex) und Crotein^{®} (Croda) erhältlich.

Einsetzbar sind auch kationisierte Proteinhydrolysate, wobei das zugrunde liegende Proteinhydrolysat vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder von biotechnologisch gewonnenen Proteinhydrolysaten, stammen kann. Die den kationischen Derivaten zugrunde liegenden Proteinhydrolysate können aus den entsprechenden Proteinen durch eine chemische, insbesondere alkalische oder saure Hydrolyse, durch eine enzymatische Hydrolyse und/oder einer Kombination aus beiden Hydrolysearten gewonnen werden. Die Hydrolyse von Proteinen ergibt in der Regel ein Proteinhydrolysat mit einer Molekulargewichtsverteilung von etwa 100 Dalton bis hin zu mehreren tausend Dalton. Bevorzugt sind solche kationischen Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Die Quaternisierung der Proteinhydrolysate oder der Aminosäuren wird häufig mittels quarternären Ammoniumsalzen wie beispielsweise N,N-Dimethyl-N-(n-Alkyl)-N-(2-hydroxy-3-chloro-n-propyl)-ammoniumhalogeniden durchgeführt. Weiterhin können die kationischen Proteinhydrolysate auch noch weiter derivatisiert sein. Als typische Beispiele für die kationischen Proteinhydrolysate und -derivate seien die unter den INCI - Bezeichnungen bekannten und im Handel erhältlichen Produkte genannt: Cocodimonium Hydroxypropyl Hydrolyzed Collagen, Cocodimopnium Hydroxypropyl Hydrolyzed Casein, Cocodimonium Hydroxypropyl Hydrolyzed Collagen, Cocodimonium Hydroxypropyl Hydrolyzed Hair Keratin, Cocodimonium Hydroxypropyl Hydrolyzed Keratin, Cocodimonium Hydroxypropyl Hydrolyzed Rice Protein, Cocodimonium Hydroxypropyl Hydrolyzed Silk, Cocodimonium Hydroxypropyl Hydrolyzed Soy Protein, Cocodimonium Hydroxypropyl Hydrolyzed Wheat Protein, Cocodimonium Hydroxypropyl Silk Amino Acids, Hydroxypropyl Arginine Lauryl/Myristyl Ether HCl, Hydroxypropyltrimonium Gelatin, Hydroxypropyltrimonium Hydrolyzed Casein, Hydroxypropyltrimonium Hydrolyzed Collagen, Hydroxypropyltrimonium Hydrolyzed Conchiolin Protein, Hydroxypropyltrimonium Hydrolyzed keratin, Hydroxypropyltrimonium Hydrolyzed Rice Bran Protein, Hydroxyproypltrimonium Hydrolyzed Silk, Hydroxypropyltrimonium Hydrolyzed Soy Protein, Hydroxypropyl Hydrolyzed Vegetable Protein, Hydroxypropyltrimonium Hydrolyzed Wheat Protein, Hydroxypropyltrimonium Hydrolyzed Wheat Protein/Siloxysilicate, Laurdimonium Hydroxypropyl Hydrolyzed Soy Protein, Laurdimonium Hydroxypropyl Hydrolyzed Wheat Protein, Laurdimonium Hydroxypropyl Hydrolyzed Wheat Protein/Siloxysilicate, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Silk, Lauryldimonium Hydroxypropyl Hydrolyzed Soy Protein, Steardimonium Hydroxypropyl Hydrolyzed Casein, Steardimonium Hydroxypropyl Hydrolyzed Collagen, Steardimonium Hydroxypropyl Hydrolyzed Keratin, Steardimonium Hydroxypropyl Hydrolyzed Rice Protein, Steardimonium Hydroxypropyl Hydrolyzed Silk, Steardimonium Hydroxypropyl Hydrolyzed Soy Protein, Steardimonium Hydroxypropyl Hydrolyzed Vegetable Protein, Steardimonium Hydroxypropyl Hydrolyzed Wheat Protein, Steartrimonium Hydroxyethyl Hydrolyzed Collagen, Quaternium-76 Hydrolyzed Collagen, Quaternium-79 Hydrolyzed Collagen, Quaternium-79 Hydrolyzed Keratin, Quaternium-79 Hydrolyzed Milk Protein, Quaternium-79 Hydrolyzed Silk, Quaternium-79 Hydrolyzed Soy Protein, Quaternium-79 Hydrolyzed Wheat Protein. Ganz besonders bevorzugt sind die kationischen Proteinhydrolysate und -derivate auf pflanzlicher Basis. Die Proteinhydrolysate und deren Derivate werden bevorzugt in Mengen von 0,01 - 10 Gew.-%, bezogen auf das gesamte Mittel (i), eingesetzt. Mengen von 0,1 bis 5 Gew.-%, insbesondere 0,1 bis 3 Gew.-%, sind ganz besonders bevorzugt.

Ebenfalls als vorteilhaft hat sich die Kombination der Zusammensetzung (i) mit Vitaminen, Provitaminen und Vitaminvorstufen sowie deren Derivaten erwiesen.

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen beispielsweise Vitamin A-Säure und deren Ester, Vitamin A-Aldehyd und Vitamin A-Alkohol sowie dessen Ester wie das Palmitat und das Acetat in Betracht. Die Vitamin A-Komponente wird bevorzugt in Mengen von 0,05-1 Gew.-%, bezogen auf die gesamte Zubereitung, eingesetzt.

Zur Vitamin B-Gruppe oder zu dem Vitamin B-Komplex gehören u. a.
- Vitamin B₁ (Thiamin)
- Vitamin B₂ (Riboflavin)
- Vitamin B₃. Unter dieser Bezeichnung werden häufig die Verbindungen Nicotinsäure und Nicotinsäureamid (Niacinamid) geführt. Bevorzugt ist das Nicotinsäureamid, das in bevorzugt in Mengen von 0,05 bis 1 Gew.-%, bezogen auf das gesamte Mittel (i), eingesetzt wird.
- Vitamin B₅ (Pantothensäure und Panthenol). Im Rahmen dieser Gruppe wird bevorzugt das Panthenol eingesetzt. Einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie kationische Panthenolderivate. Die genannten Verbindungen des Vitamin B₅-Typs werden bevorzugt in Mengen von 0,05 - 10 Gew.-%, bezogen auf das gesamte Mittel (i), eingesetzt. Mengen von 0,1 - 5 Gew.-% sind besonders bevorzugt.
- Vitamin B₆ (Pyridoxin sowie Pyridoxamin und Pyridoxal).

Vitamin C (Ascorbinsäure). Die übliche Einsatzmenge von Vitamin C beträgt 0,1 bis 3 Gew.-%, bezogen auf das gesamte Mittel (i). Die Verwendung in Form des Palmitinsäureesters, der Glucoside oder Phosphate kann bevorzugt sein. Die Verwendung in Kombination mit Tocopherolen kann ebenfalls bevorzugt sein.

Vitamin E (Tocopherole, insbesondere α-Tocopherol). Tocopherol und seine Derivate, worunter insbesondere die Ester wie das Acetat, das Nicotinat, das Phosphat und das Succinat fallen, werden bevorzugt in Mengen von 0,05-1 Gew.-%, bezogen auf das gesamte Mittel (i), eingesetzt. Vitamin F. Unter dem Begriff "Vitamin F" werden üblicherweise essentielle Fettsäuren, insbesondere Linolsäure, Linolensäure und Arachidonsäure, verstanden.

Vitamin H. Als Vitamin H hat sich der Trivialname Biotin durchgesetzt. Biotin wird bevorzugt in Mengen von 0,0001 bis 1,0 Gew.-%, insbesondere in Mengen von 0,001 bis 0,01 Gew.-% eingesetzt.

Besonders bevorzugt ist der Einsatz von Vitaminen, Provitaminen und Vitaminvorstufen aus den Gruppen A, B, E und H in den Reinigungsmitteln (i), wobei Panthenol, Pantolacton und Nicotinsäureamid besonders bevorzugt sind.

Eine weitere fakultative Komponente ist ein Pflanzenextrakt.

Unter geeigneten Pflanzenextrakten sind Extrakte zu verstehen, die aus allen Teilen einer Pflanze hergestellt werden können.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Bevorzugt sind vor allem die Extrakte aus Grünem Tee, Eichenrinde, Brennessel, Hamamelis, Hopfen, Kamille, Klettenwurzel, Schachtelhalm, Weißdorn, Lindenblüten, Litschi, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Ginseng, Ingwerwurzel, Echinacea purpurea, Olea europea, Foeniculum vulgaris und Apim graveolens.

Als Extraktionsmittel zur Herstellung der genannten Pflanzenextrakte können Wasser, Alkohole sowie deren Mischungen verwendet werden. Unter Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.

Die Pflanzenextrakte können sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch.

Zusätzlich kann es sich als vorteilhaft erweisen, wenn die Reinigungsmittel (i) Feuchthaltemittel bzw. Penetrationshilfsstoffe und/ oder Quellmittel (M) enthalten. Diese Hilfsstoffe sorgen für eine bessere Penetration von Wirkstoffen in die keratinische Faser oder helfen die keratinische Faser aufzuquellen. Hierzu sind beispielsweise zu zählen Harnstoff und Harnstoffderivate, Guanidin und dessen Derivate, Arginin und dessen Derivate, Wasserglas, Imidazol und dessen Derivate, Histidin und dessen Derivate, Benzylalkohol, Glycerin, Carbonate, Hydrogencarbonate, Diole und Triole, und insbesondere 1,2-Diole und 1,3-Diole wie beispielsweise 1,2-Propandiol, 1,2-Pentandiol, 1,2-Hexandiol, 1,2-Dodecandiol, 1,3-Propandiol, 1,6-Hexandiol, 1,5-Pentandiol, 1,4-Butandiol. Besonders geeignet ist Glycerin.

Die Feuchthaltemittel und/oder Quellmittel werden in den Zusammensetzungen (i) - bezogen auf die gesamte Zusammensetzung - bevorzugt in Mengen von 0,05 bis 5 Gew.-% und insbesondere in Mengen von 0,1 bis 3 Gew.-% eingesetzt.

Weiterhin können die Reinigungsmittel (i) in einer bevorzugten Ausführungsform zusätzlich einen UV - Filter (I) enthalten. Die einsetzbaren UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt.

Die UV-Filter können beispielsweise ausgewählt werden aus substituierten Benzophenonen, p-Aminobenzoesäureestern, Diphenylacrylsäureestern, Zimtsäureestern, Salicylsäureestern, Benzimidazolen und o-Aminobenzoesäureestern.

Gemäß einer weiteren Ausführungsform sind solche UV-Filter bevorzugt, die eine kationische Gruppe, insbesondere eine quartäre Ammoniumgruppe, aufweisen.

Zwei bevorzugte UV-Filter mit kationischen Gruppen sind die als Handelsprodukte erhältlichen Verbindungen Zimtsäureamidopropyl-trimethylammoniumchlorid (Incroquat^{®}UV-283) und Dodecyldimethylaminobenzamidopropyl-dimethylammoniumtosylat (Escalol^{®} HP 610).

Der oder die UV-Filter wird (werden) üblicherweise in Mengen 0,1-5 Gew.-%, bezogen auf das gesamte Mittel (i), eingesetzt. Mengen von 0,4-2,5 Gew.-% sind bevorzugt.

Der pH-Wert der Reinigungsmittel (i) liegt bevorzugt in einem schonenden Bereich für die Haut von etwa 2,5 bis 6,5 und insbesondere in einem Bereich von 3 bis 6.

Neben den zuvor genannten Komponenten können prinzipiell alle weiteren, dem Fachmann für solche kosmetischen Mittel bekannten Komponenten eingesetzt werden.

Solche weiteren Wirk-, Hilfs- und Zusatzstoffe sind beispielsweise:
- Strukturanten wie Maleinsäure und Milchsäure,
- Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- Farbstoffe zum Anfärben des Mittels,
- Antischuppenwirkstoffe wie Piroctone Olamine, Zink Omadine und Climbazol,
- weitere Substanzen zur Einstellung des pH-Wertes, wie beispielsweise α- und β -Hydroxycarbonsäuren,
- Wirkstoffe wie Allantoin und Bisabolol,
- Komplexbildner wie EDTA, NTA, β-Alanindiessigsäure und Phosphonsäuren,
- Perlglanzmittel wie Ethylenglykolmono- und -distearat sowie PEG-3-distearat,
- Pigmente,
- Konsistenzgeber wie Zuckerester, Polyolester oder Polyolalkylether,
- Pigmente,
- Konservierungsmittel, wie beispielsweise Natriumbenzoat oder Salicylsäure,
- Viskositätsregler wie Salze (NaCl).

Ein zweiter Gegenstand der Erfindung ist ein Verfahren zur Reinigung von Haaren, bei dem ein kosmetisches Reinigungsmittel (i) aus dem erfindungsgemäßen Reinigungsprodukt als feiner Nebel auf den Haaren verteilt, mit Wasser aufgeschäumt, einmassiert und nach einer Einwirkungszeit von mindestens 30 Sekunden mit Wasser wieder ausgespült wird.

Der Vorteil des erfindungsgemäßen Reinigungsprodukts besteht darin, dass die Reinigungsmittel (i) besonders mild sind und ihre Applikationsform eine besonders einfache Handhabbarkeit gewährleistet, denn das Reinigungsmittel (i) kann durch einfaches Pumpen oder Drücken direkt auf die vorzugsweise nasse Anwendungsoberfläche gebracht werden, ohne vorher mit den Händen in Berührung zu kommen.

Ein weiterer Vorteil besteht darin, dass das Reinigungsmittel (i) als feiner Sprühnebel auf die Anwendungsoberfläche gebracht werden kann, wodurch sich eine bessere Verteilung der Reinigungszubereitung (i) erreichen lässt, denn die feinen Tröpfchen erreichen jeden Bereich der Anwendungsoberfläche.

Das erfindungsgemäße Reinigungsprodukt erlaubt außerdem einen sparsamen Gebrauch, was aus Kosten- und Umweltgründen ebenfalls von Vorteil ist.

Schließlich besteht ein weiterer Vorteil der vorliegenden Erfindung darin, dass die Herstellung des Reinigungsmittels (i) sich signifikant vereinfacht, denn gegebenenfalls anwesende, nicht in Wasser lösliche haut- und haarkonditionierende Komponenten müssen nicht durch zusätzliche Wirkstoffe in dem Reinigungsmittel stabilisiert werden (beispielsweise um eine klare Formulierung oder eine stabile Emulsion oder Dispersion zu bilden), sondern werden dem Mittel nach Bedarf einfach zugegeben.

Ebenso muss das Reinigungsmittel (i) nicht zusätzlich verdickt werden, um leichter handhabbar zu werden (d.h. um eine gleichmäßige Verteilung aller Wirkstoffe in dem Reinigungsmittel zu erreichen und um eine Konsistenz des Mittels zu schaffen die es erlaubt, dass der Anwender das Mittel leicht aus dem Behälter entnehmen und auf die Haare auftragen kann, ohne dass ihm das Reinigungsmittel zwischen den Fingern durchläuft).

Eine gleichmäßige Verteilung aller notwendiger und in dem Mittel (i) erwünschter Komponenten lässt sicht vielmehr dadurch erzielen, dass der Spender (ii) mit der Reinigungszusammensetzung vor der Verwendung kräftig geschüttelt, und das Reinigungsmittel (i) unmittelbar danach als feiner Sprühnebel aus dem Spender auf die Anwendungsoberfläche gebracht wird. Durch das Schütteln werden alle Komponenten kurzzeitig miteinander vermischt und können zusammen auf die - vorzugsweise nasse - Anwendungsoberfläche gesprüht werden. Dort werden sie einmassiert, wodurch sie wie ein übliches Reinigungsmittel aufschäumen. Nach dem Reinigungsvorgang werden sie mit Wasser wieder ausgespült.

### Beispiele für erfindungsgemäße Shampoos

| Rohstoff | **1** | **2** |
|---|---|---|
| Plantacare^{®1} 818 UP | 10,00 | 9,00 |
| Plantapon^{®2} ACG 50 | 8,00 | 7,50 |
| Rewoteric^{®3} AM C | 10,00 | 8,00 |
| Salicylsäure | 0,20 | 0,20 |
| Polymer^{®4} JR 400 | 0,50 | 0,50 |
| Panthenol | 0,20 | - |
| Nicotinsäureamid | - | 0,15 |
| Hydrolyzed Wheat Protein | - | 0,10 |
| Natriumbenzoat | 0,50 | 0,50 |
| Citronensäure | 1,60 | 1,55 |
| Parfum | 0,30 | 0,30 |
| Sonnenblumenöl | - | 0,20 |
| Aprikosenkernöl | 0,10 | - |
| Konservierungsmittel | q.s. | q.s. |
| Wasser | ad 100 | ad 100 |

Es wurden die folgenden Handelsprodukte eingesetzt:
- 1: INCl-Bezeichnung: Coco-Glucoside (Aktivsubstanz: etwa 50%), Cognis
- 2: INCl-Bezeichnung: Disodium Cocoyl Glutamate (Aktivsubstanz: etwa 50%), Cognis
- 3: INCl-Bezeichnung: Sodium Cocoamphoacetate (Aktivsubstanz: etwa 40%), Evonik Degussa
- 4: INCl-Bezeichnung: Polymquaternium-10; Dow

Die Reinigungszusammensetzungen 1 und 2 wurden in einen Pumpzerstäuber PZ 2 / 190 DL 20/410 der Firma Seaquist Perfect Dispensing GmbH mit einem Sprühkopf des Typs PSK 001 (Seaquist) abgefüllt.

Vor der Anwendung wurden die Reinigungszubereitungen geschüttelt, um eine kurzzeitige Durchmischung der Inhaltsstoffe zu erreichen, und unmittelbar danach als feiner Sprühnebel auf - vorzugsweise nasse - Haare gesprüht. Das Shampoo wurde mit Wasser aufgeschäumt, einmassiert und nach etwa 1 Minute wieder ausgespült.

## Patentansprüche

1. Sensitives kosmetisches Reinigungsprodukt, umfassend
(i) ein sensitives kosmetisches Reinigungsmittel, enthaltend
a) mindestens ein mildes anionisches Tensid, das ein unter der INCI-Bezeichnung Disodium Cocoyl Glutamate bekanntes anionisches Tensid enthält,
b) mindestens ein amphoteres/zwitterionisches Tensid, und
c) mindestens ein nichtionisches Tensid
in einem Verhältnis der Tenside a), b) und c) im Bereich von (1-2) : (0,5-1) : (1-2), und
(ii) einen Spender zur Bevorratung und Abgabe des Reinigungsmittels (i), der mindestens einen Behälter zur Bevorratung des Reinigungsmittels, eine Abgabevorrichtung zur Abgabe des Reinigungsmittels in gesprühter Form und eine Fördereinrichtung zur Förderung des Reinigungsmittels vom Behälter zur Abgabevorrichtung aufweist,
wobei sich das Verhältnis auf das Gewichtsverhältnis aller milder anionischen Tenside a), aller amphoterer/zwitterionischer Tenside b) und aller nichtionischer Tenside c) bezieht.

2. Reinigungsprodukt nach Anspruch 1, **dadurch gekennzeichnet, dass** das Reinigungsmittel (i) eine Viskosität von maximal 1000 mPas, bevorzugt von maximal 750 mPas und insbesondere von maximal 500 mPas aufweist (gemessen mit einem Haake-Viskosimeter NV bei 20°C und 8UpM).

3. Reinigungsprodukt nach einem der Ansprüche 1 oder 2, **dadurch gekennzeichnet, dass** das Reinigungsmittel (i) frei von Verbindungen und/oder Gruppierungen der folgenden Formeln ist:
a) H-(O-CH₂CH₂)ₙ-OH, in der n für ganze Zahlen zwischen 1 und 100.000 steht,
b) H-(O-CH(CH₃)CH₂)ₙ-OH, in der n für ganze Zahlen zwischen 1 und 100.000 steht,
c) R-(O-CH₂CH₂)ₙ-OH, in der R für einen geradkettigen oder verzweigten, gesättigten oder ungesättigten Alkyl- oder Alkenylrest mit 1 bis 30 C-Atomen, und n für ganze Zahlen zwischen 1 und 10.000 steht,
d) R-(O-CH₂CH₂)ₙOSO₃H, in der R für einen geradkettigen oder verzweigten, gesättigten oder ungesättigten Alkyl- oder Alkenylrest mit 1 bis 30 C-Atomen, und n für ganze Zahlen zwischen 1 und 10.000 steht, und
e) -(O-CH₂CH₂)ₙ-, in der n für ganze Zahlen zwischen 2 und 100.000 steht.

4. Reinigungsprodukt nach einem der Ansprüche 1 bis 3, **dadurch gekennzeichnet, dass** das Reinigungsmittel (i) frei von anionischen Sulfattensiden und frei von polymeren Verdickungsmitteln ist.

5. Reinigungsprodukt nach einem der Ansprüche 1 bis 4, **dadurch gekennzeichnet, dass** das Reinigungsmittel (i)
- mindestens ein amphoteres/zwitterionisches Tensid b) aus der Gruppe der N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren mit jeweils etwa 8 bis 24 C-Atomen in der Alkylgruppe, Alkylaminoessigsäuren mit jeweils etwa 8 bis 24 C-Atomen in der Alkylgruppe, N-Kokosalkylaminopropionat, Kokosacylaminoethylaminopropionat, C₁₂-C₁₈ - Acylsarcosin, N-Alkyl-N,N-dimethylammonium-glycinate, beispielsweise Kokosalkyl-dimethylammoniumglycinat, N-Acyl-aminopropyl-N,N-dimethylammoni umglycinate, beispielsweise Kokosacylaminopropyl-dimethylammoniumglycinat, 2-Alkyl-3-carboxymethyl-3-hydroxyethyl-imidazoline mit jeweils 8 bis 18 C-Atomen in der Alkyl- oder Acylgruppe, Kokosacylaminoethylhydroxyethylcarboxymethyl glycinat, der unter der INCI-Bezeichnung Cocamidopropyl Betain bekannten Verbindungen, der unter der INCI-Bezeichnung Disodium Cocoamphodiacetate bekannten Verbindungen und
- mindestens ein nichtionisches Tensid c) aus der Gruppe der Alkylpolyglucoside enthält.

6. Reinigungsprodukt nach einem der Ansprüche 1 bis 5, **dadurch gekennzeichnet, dass** das Reinigungsmittel (i) - bezogen auf sein Gesamtgewicht - zusätzlich 0,01 bis 3 Gew.-% mindestens eines kationischen Polymeren enthält, das ausgewählt ist aus kationischen Cellulosepolymeren, kationischen Guarpolymeren, kationischen Polymeren auf Acrylsäure(derivat)basis sowie aus Gemischen dieser Polymertypen.

7. Reinigungsprodukt nach einem der Ansprüche 1 bis 6, **dadurch gekennzeichnet, dass** das Reinigungsmittel (i) - bezogen auf sein Gesamtgewicht - zusätzlich 0,005 bis 10 Gew.-%, bevorzugt 0,01 bis 5 Gew.-% und insbesondere 0,05 bis 3 Gew.-% mindestens einer weiteren Pflegekomponente aus der Gruppe der Öl-, Wachs- und/oder Fettkomponenten enthält, die ausgewählt sind aus mineralischen, natürlichen oder synthetischen Öl-, Wachs- und/oder Fettkomponenten wie Petrolatum, Paraffin, Silikonen, Fettalkoholen, Fettsäuren, Fettsäureestern sowie natürlichen Ölen und Wachsen pflanzlichen und tierischen Ursprungs.

8. Kosmetisches Verfahren zur Reinigung von Haaren, **dadurch gekennzeichnet, dass** ein kosmetisches Reinigungsmittel aus einem Reinigungsprodukt nach einem der Ansprüche 1 bis 8 als feiner Nebel auf den Haaren verteilt, mit Wasser aufgeschäumt, einmassiert und nach einer Einwirkungszeit von mindestens 30 Sekunden mit Wasser wieder ausgespült wird.

## Claims

1. Sensitive cosmetic cleaning product, encompassing
(i) a sensitive cosmetic cleaning agent, comprising
a) at least one mild anionic surfactant that comprises an anionic surfactant known by the INCI name Disodium Cocoyl Glutamate,
b) at least one amphoteric/zwitterionic surfactant, and
c) at least one non-ionic surfactant
wherein the ratio of the surfactants a), b) and c) is in the range (1-2):(0.5-1):(1-2), and
(ii) a dispenser for storing and dispensing the cleaning agent (i) and which possesses at least one vessel for storing the cleaning agent, one dispensing device for dispensing the cleaning agent in the form of a spray and one conveying device for conveying the cleaning agent from the vessel to the dispensing device,
wherein the ratio refers to the weight ratio of all the mild anionic surfactants a), all the amphoteric/zwitterionic surfactants b) and all the non-ionic surfactants c).

2. Cleaning product according to claim 1, **characterised in that** the cleaning agent (i) has a viscosity of maximum 1000 mPas, preferably maximum 750 mPas and in particular maximum 500 mPas (measured with a Haake viscosimeter NV at 20°C and 8 rpm).

3. Cleaning product according to one of claims 1 or 2, **characterised in that** the cleaning agent (i) is free of compounds and/or groups of the following formulae:
a) H-(O-CH₂CH₂)ₙ-OH, in which n stands for whole numbers between 1 and 100 000,
b) H-(O-CH(CH₃)CH₂)ₙ-OH, in which n stands for whole numbers between 1 and 100 000,
c) R-(O-CH₂CH₂)ₙ-OH, in which R stands for a straight chain or branched, saturated or unsaturated alkyl or alkenyl group with 1 to 30 carbon atoms, and n stands for whole numbers between 1 and 10 000,
d) R-(O-CH₂CH₂)ₙ-OSO₃H, in which R stands for a straight chain or branched, saturated or unsaturated alkyl or alkenyl group with 1 to 30 carbon atoms, and n stands for whole numbers between 1 and 10 000, and
e) -(O-CH₂CH₂)ₙ-, in which n stands for whole numbers between 2 and 100 000.

4. Cleaning product according to one of claims 1 to 3, **characterised in that** the cleaning agent (i) is free of anionic sulfate surfactants and free of polymeric thickeners.

5. Cleaning product according to one of claims 1 to 4, **characterised in that** the cleaning agent (i) comprises
- at least one amphoteric/zwitterionic surfactant b) from the group of the *N*-alkylglycines, *N*-alkylpropionic acids, *N*-alkylamino butyric acids, *N*-alkylimino dipropionic acids, *N*-hydroxyethyl-N-alkylamidopropylglycines, *N*-alkyltaurines, *N*-alkylsarcosines, 2-alkylamino propionic acids each containing about 8 to 24 carbon atoms in the alkyl group, alkylamino acetic acids each containing about 8 to 24 carbon atoms in the alkyl group, *N*-cocoalkylamino propionate, cocoacylaminoethylamino propionate, C₁₂ - C₁₈ acylsarcosine, *N*-alkyl-*N*,*N*-dimethylammonium glycinates, for example cocoalkyldimethylammonium glycinate, *N*-acyl-aminopropyl-*N*,*N*-dimethylammonium glycinates, for example cocoacylaminopropyldimethylammonium glycinate, 2-alkyl-3-carboxymethyl-3-hydroxyethyl-imidazolines, each containing about 8 to 18 carbon atoms in the alkyl or acyl group, cocoacylaminoethylhydroxyethylcarboxymethyl glycinate, the compounds known by the INCI name Cocamidopropyl Betaine, the compounds known by the INCI name Disodium Cocoamphodiacetate and
- at least one non-ionic surfactant c) from the group of the alkyl polyglucosides.

6. Cleaning product according to one of claims 1 to 5, **characterised in that** the cleaning agent (i) - based on its total weight - additionally comprises 0.01 to 3 wt % of at least one cationic polymer that is selected from cationic cellulosic polymers, cationic guar polymers, cationic polymers based on acrylic acid (derivatives) as well as mixtures of these polymer types.

7. Cleaning product according to one of claims 1 to 6, **characterised in that** the cleaning agent (i) - based on its total weight - additionally comprises 0.005 to 10 wt %, preferably 0.01 to 5 wt % and in particular 0.05 to 3 wt % of at least one additional care component from the group of the oily, waxy and/or fatty components such as petrolatum, paraffin, silicones, fatty alcohols, fatty acids, fatty acid esters as well as natural oils and waxes of vegetal and animal origin.

8. Cosmetic method for cleaning hair, **characterised in that** a cosmetic cleaning agent of a cleaning product according to one of claims 1 to 8 is dispersed as a fine mist onto the hair, is foamed with water, is massaged in and after a contact time of at least 30 seconds is rinsed out again with water.

## Revendications

1. Produit de nettoyage cosmétique sensitif, comprenant
(i) un agent de nettoyage cosmétique sensitif, contenant
a) au moins un agent tensioactif anionique doux, qui contient un agent tensioactif anionique connu sous la dénomination INCI Disodium Cocoyl Glutamate,
b) au moins un agent tensioactif amphotère/zwittérionique, et
c) au moins un agent tensioactif non ionique
dans un rapport des agents tensioactifs a), b) et c) dans la plage de (1-2):(0,5-1):(1-2), et
(ii) un distributeur pour le stockage et la distribution de l'agent de nettoyage (i), qui présente au moins un récipient pour le stockage de l'agent de nettoyage, un dispositif de distribution pour la distribution du produit de nettoyage sous forme pulvérisée et un dispositif de transport pour le transport de l'agent de nettoyage du récipient vers le dispositif de distribution,
le rapport se rapportant au rapport pondéral de tous les agents tensioactifs a) anioniques doux, tous les agents tensioactifs b) amphotères/zwittérioniques et tous les agents tensioactifs c) non ioniques.

2. Produit de nettoyage selon la revendication 1, **caractérisé en ce que** l'agent de nettoyage (i) présente une viscosité d'au maximum 1000 mPa.s, de préférence d'au maximum 750 mPa.s et en particulier d'au maximum 500 mPa.s (mesurée à l'aide d'un viscosimètre de Haake NV à 20°C et à 8 t/min).

3. Produit de nettoyage selon l'une quelconque des revendications 1 ou 2, **caractérisé en ce que** l'agent de nettoyage (i) est exempt de composés et/ou de groupements des formules suivantes :
a) H-(O-CH₂CH₂)ₙ-OH, dans laquelle n représente des nombres entiers entre 1 et 100 000,
b) H-(O-CH(CH₃)CH₂)ₙ-OH, dans laquelle n représente des nombres entiers entre 1 et 100 000,
c) R-(O-CH₂CH₂)ₙ-OH, dans laquelle R représente un résidu alkyle ou alcényle linéaire ou ramifié, saturé ou insaturé, comprenant 1 à 30 atomes de carbone et n représente des nombres entiers entre 1 et 10 000,
d) R-(O-CH₂CH₂)ₙ-OSO₃H, dans laquelle R représente un résidu alkyle ou alcényle linéaire ou ramifié, saturé ou insaturé, comprenant 1 à 30 atomes de carbone et n représente des nombres entiers entre 1 et 10 000, et
e) -(O-CH₂CH₂)ₙ-, dans laquelle n représente des nombres entiers entre 2 et 100 000.

4. Produit de nettoyage selon l'une quelconque des revendications 1 à 3, **caractérisé en ce que** l'agent de nettoyage (i) est exempt d'agents tensioactifs sulfatés anioniques et exempt d'épaississants polymères.

5. Produit de nettoyage selon l'une quelconque des revendications 1 à 4, **caractérisé en ce que** l'agent de nettoyage (i) contient
- au moins un agent tensioactif b) amphotère/zwittérionique du groupe formé par les N-alkylglycines, les acides N-alkylpropioniques, les acides N-alkylaminobutyriques, les acides N-alkyliminodipropioniques, les N-hydroxyéthyl-N-alkylamidopropylglycines, les N-alkyltaurines, les N-alkylsarcosines, les acides 2-alkylaminopropioniques comprenant à chaque fois environ 8 à 24 atomes de carbone dans le groupe alkyle, les acides alkylaminoacétiques comprenant à chaque fois environ 8 à 24 atomes de carbone dans le groupe alkyle, le N-coco-alkylaminopropionate, le coco-acylaminoéthylaminopropionate, la C₁₂-C₁₈-acylsarcosine, les glycinates de N-alkyl-N,N-diméthylammonium, par exemple le glycinate de coco-alkyldiméthylammonium, les glycinates de N-acylaminopropyl-N,N-diméthylammonium, par exemple le glycinate de coco-acylaminopropyldiméthylammonium, les 2-alkyl-3-carboxyméthyl-3-hydroxyéthylimidazolines comprenant à chaque fois 8 à 18 atomes de carbone dans le groupe alkyle ou acyle, le coco-acylaminoéthylhydroxyéthylcarboxyméthylglycinate, les composés connus sous la dénomination INCI "Cocamidopropyl Betain", les composés connus sous la dénomination INCI "Disodium Cocoamphodiacetate" et
- au moins un agent tensioactif c) non ionique du groupe formé par les alkylpolyglucosides.

6. Produit de nettoyage selon l'une quelconque des revendications 1 à 5, **caractérisé en ce que** l'agent de nettoyage (i) contient en outre - par rapport à son poids total - 0,01 à 3% en poids d'au moins un polymère cationique, qui est choisi parmi les polymères de cellulose cationiques, les polymères de guar cationiques, les polymères cationiques à base (d'un dérivé) d'acide acrylique ainsi que les mélanges de ces types de polymères.

7. Produit de nettoyage selon l'une quelconque des revendications 1 à 6, **caractérisé en ce que** l'agent de nettoyage (i) contient en outre - par rapport à son poids total - 0,005 à 10% en poids, de préférence 0,01 à 5% en poids et en particulier 0,05 à 3% en poids d'au moins un autre composant de soin du groupe formé par les composants huileux, cireux et/ou gras, qui sont choisis parmi les composants huileux, cireux et/ou gras minéraux, naturels ou synthétiques, tels que la vaseline, la paraffine, les silicones, les alcools gras, les acides gras, les esters d'acides gras ainsi que les huiles et cires naturelles d'origine végétale et animale.

8. Procédé cosmétique pour le nettoyage de cheveux, **caractérisé en ce qu'**un agent de nettoyage cosmétique provenant d'un produit de nettoyage selon l'une quelconque des revendications 1 à 8 est réparti sur les cheveux sous forme d'un brouillard fin, moussé avec de l'eau, soumis à une pénétration par massage et à nouveau éliminé par rinçage à l'eau après un temps d'action d'au moins 30 secondes.
